# EUROPEAN PATENT SPECIFICATION

(11) **EP 3 179 830 B1**
(45) Date of publication and mention of the grant of the patent: **23.09.2026**
(21) Application number: 17161724.4
(22) Date of filing: 18.03.2017
(51) Int. Cl.: H05B 6/10, A24F 40/10, A24F 40/465, A24F 40/485

(54) **HEATING ASSEMBLY, ATOMIZER AND ELECTRONIC CIGARETTE HAVING SAME**
ERWÄRMUNGSANORDNUNG, ZERSTÄUBER UND ELEKTRONISCHE ZIGARETTE DAMIT
ENSEMBLE DE CHAUFFAGE, ATOMISEUR ET CIGARETTE ÉLECTRONIQUE LES COMPRENANT

(30) Priority: 21.03.2016 CN 201620219324 U
(43) Date of publication of application: 14.06.2017
(73) Proprietor: Shenzhen First Union Technology Co., Ltd., Shenzhen, Guangdong 518104 (CN)
(72) Inventor: LI, Yonghai, Shenzhen, Guangdong 518104 (CN); XU, Zhongli, Shenzhen, Guangdong 518104 (CN); HU, Shuyun, Shenzhen, Guangdong 518104 (CN)
(74) Representative: Proi World Intellectual Property GmbH

(56) References cited:
- EP-A1- 2 460 422
- EP-A1- 2 468 117
- EP-A1- 2 965 642
- EP-A1- 3 020 292
- EP-A2- 3 153 199
- CN-A- 103 689 812
- CN-U- 204 317 492
- US-A1- 2015 245 669
- US-A1- 2015 320 116
- US-A1- 2016 073 692
- US-A1- 2017 055 583

## Description

### TECHNICAL FIELD

The present invention relates to a heating assembly, an atomizer and an electronic cigarette using same.

### BACKGROUND ART

A typical electronic cigarette includes a liquid conducting body, and a heating wire wrapped around the liquid conducting body. The heating wire is in tight contact with the liquid conducting body. Heat of the heating wire is conveyed to the liquid conducting body, and then the tobacco liquid in the liquid conducting body is heated to form aerosol. However, a thermal efficiency of the electronic cigarette is low.

What are needed, therefore, are a heating assembly, an atomizer and an electronic cigarette using same, which can overcome the above shortcomings.

EP 2 460 422 A1 discloses an aerosol generating system for heating a liquid aerosol-forming substrate, the system comprising an aerosol-forming chamber and leakage prevention means configured to prevent or reduce leakage of liquid aerosol condensate from the aerosol generating system. The leakage prevention means comprises a cavity in a wall of the aerosol-forming chamber and a hooked member for collecting droplets of condensed liquid aerosol-forming substrate, an impactor for disrupting airflow in the aerosol-forming chamber so as to collect liquid droplets, and a closure member for substantially sealing the aerosol-forming chamber when the aerosol generating system is not in use. CN 103 689 812 A discloses a smoke generator comprising a shell and a heating component, wherein the shell is provided with a smoke inlet hole and a smoke outlet hole, and the heating component is arranged in the shell. The shell is further provided with an accommodating area used for storing smoke generating materials. The heating component comprises a metal component and an induction coil wound on the periphery of the metal component.

### SUMMARY

An exemplary heating assembly includes a liquid conducting and heating body and an inductive coil corresponding to the liquid conducting and heating body. The liquid conducting and heating body includes a porous ceramic main body and a metallic powder evenly distributed in the ceramic main body. The inductive coil is configured for generating high frequency magnetic field in response to an alternating current, so that eddy current and heat are generated in the liquid conducting and heating body. The liquid conducting and heating body is configured for absorbing tobacco liquid and heating the tobacco liquid to form aerosol. The ceramic main body and the metallic powder is integrally formed.

### BRIEF DESCRIPTION OF THE DRAWINGS

Many aspects of the present invention can be better understood with reference to the following drawings. The components in the drawings are not necessarily drawn to scale, the emphasis instead being placed upon clearly illustrating the principles of the present invention. Moreover, in the drawings, like reference numerals designate corresponding parts throughout the several views.
FIG. 1 is a cross-sectional view of an electronic cigarette according to a first embodiment, including a heating assembly.
FIG. 2 is a cross-sectional view of the heating assembly of FIG. 1.
FIG. 3 is a perspective view of a heating assembly.
FIG. 4 is a perspective view of an electronic cigarette of FIG. 1.
FIG. 5 is an enlarged view of area AA of FIG. 4.
FIG. 6 is a cross-sectional view of a heating assembly according to a second embodiment.

### DETAILED DESCRIPTION

It will be appreciated that for simplicity and clarity of illustration, where appropriate, reference numerals have been repeated among the different figures to indicate corresponding or analogous elements. In addition, numerous specific details are set forth in order to provide a thorough understanding of the embodiments described herein. However, it will be understood by those of ordinary skill in the art that the embodiments described herein can be practiced without these specific details. In other instances, methods, procedures and components have not been described in detail so as not to obscure the related relevant feature being described. Also, the description is not to be considered as limiting the scope of the embodiments described herein. The drawings are not necessarily to scale and the proportions of certain parts have been exaggerated to better illustrate details and features of the present invention.

The invention is illustrated by way of example and not by way of limitation in the figures of the accompanying drawings in which like references indicate similar elements. It should be noted that references to "an" or "one" embodiment in this invention are not necessarily to the same embodiment, and such references mean at least one.

Several definitions that apply throughout this invention will now be presented.

The term "outside" refers to a region that is beyond the outermost confines of a physical object. The term "inside" indicates that at least a portion of a region is partially contained within a boundary formed by the object. The term "substantially" is defined to be essentially conforming to the particular dimension, shape or other word that substantially modifies, such that the component need not be exact. For example, substantially cylindrical means that the object resembles a cylinder, but can have one or more deviations from a true cylinder. The term "comprising," when utilized, means "including, but not necessarily limited to"; it specifically indicates open-ended inclusion or membership in the so-described combination, group, series and the like.

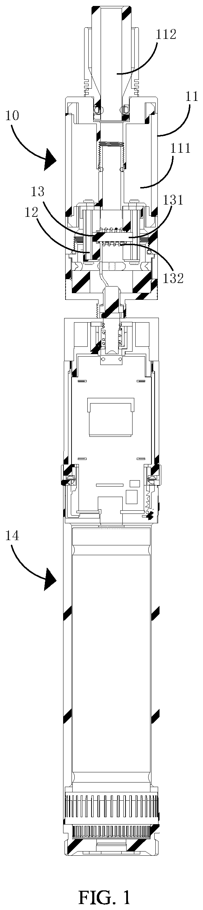

Referring to FIGS. 1-2, an electronic cigarette includes a main body 11, a liquid conducting groove 12, a heating assembly 13, and a power supply 14. The power supply 14 includes a rechargeable battery and a high frequency DC/AC inverter. The DC/AC inverter is configured (i.e., structured and arranged) for converting direct current to high frequency alternating current, and feeding the alternating current to the heating assembly 13. The main body defines a liquid chamber 111 and an air passage 112. The liquid chamber 111 is configured for storing tobacco liquid. The heating assembly 13 includes a liquid conducting and heating body 131 and an inductive coil 132.

The electronic cigarette includes two parts: a top part as the atomizer 10 and a bottom part (i.e., the power supply 14). The atomizer 10 and the power supply 14 are coupled with each, and are electrically connected via electrodes. The liquid chamber 111 is defined in a housing of the atomizer 10. The heating assembly 13 is detachably arranged in the housing. It is to be noted that, in other embodiments, the atomizer 10 and the power supply 14 may be integrally formed.

The heating assembly 13 is arranged on the air passage 112. The liquid conducting and heating body 131 is in communication with the liquid chamber 111 via the liquid conducting groove 12. The liquid conducting and heating body 131 generates aerosol from the tobacco liquid, and then the aerosol is outputted via the air passage 112

In detail, the liquid conducting groove 12 and the air passage 112 are oriented along an axial direction of the main body 11, and the liquid conducting and heating body 131 is oriented substantially perpendicular to the liquid conducting groove 12 and the air passage 112. Ends of the liquid conducting and heating body 131 are plugged into the liquid conducting groove 12, and are in contact with the tobacco liquid in the liquid conducting groove 12.

In the present embodiment, quite usefully, the liquid conducting groove 12 includes two substantially parallel liquid conducting sub-grooves 121. The liquid conducting and heating body 131 is substantially arranged between the two liquid conducting sub-grooves 121. Two opposite ends of the liquid conducting and heating body 131 are inserted into the two liquid conducting sub-grooves 121, respectively.

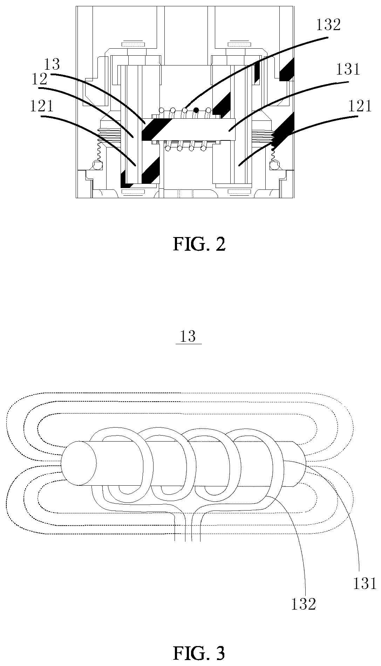

Referring to FIG. 3 together, the heating assembly 13 includes the liquid conducting and heating body 131 and the inductive coil 132. The inductive coil 132 is configured for generating high frequency magnetic field so that the liquid conducting and heating body 131 generates eddy current and heat. The liquid conducting and heating body 131 is configured for absorbing tobacco liquid and heating the tobacco liquid to form aerosol.

In the present embodiment, the liquid conducting and heating body 131 may be cylindrical or tubular. That is, the liquid conducting and heating body 131 may be a solid cylinder or a hollow cylinder.

In detail, the liquid conducting and heating body 131 includes a porous ceramic main body (not shown) and metallic powder (not shown) evenly distributed in the ceramic main body, and the ceramic main body and the metallic powder is integrally formed. Due to a porous structure of the liquid conducting and heating body 131, the tobacco liquid permeates from ends of the liquid conducting and heating body 131 to a middle portion via a capillary action. A permeating speed of the tobacco liquid can be controlled by adjusting the porosity of the liquid conducting and heating body 131 during process of manufacturing the liquid conducting and heating body 131. Quite usefully, the ceramic body may be made of at least one of aluminum oxide, zirconium oxide, kaolin, diatomite, montmorillonite; the metallic power is iron cobalt powder.

The liquid conducting and heating body 131 is made using a method described below. First, a mixture is made by uniformly mixing a metallic powder (e.g., iron cobalt powder), foaming agent (e.g., polyurethane foaming agent), and at least one of aluminum oxide, zirconium oxide, kaolin, diatomite, and montmorillonite. Then, the mixture is molded to a predetermined shape (e.g., a cylindrical structure) using a mold. Finally, the molded mixture is under heat treatment to remove the foaming agent, thus obtaining the liquid conducting and heating body. The process of heat treatment includes the following steps: naturally withering the molded mixture; sintering the molded mixture in a high temperature furnace in a predetermined temperature. Quite usefully, the predetermined temperature is 1200-1400 Celsius Degrees.

In the present embodiment, the conductive coil 132 is wound around an outer peripheral surface of the liquid conducting and heating body 131. The conductive coil 132 is adjacent to the liquid conducting body 11, but not in contact with the liquid conducting and heating body 11. The inductive coil 132 is insulated from the liquid conducting and heating body 131. To achieve insulation, the inductive coil 132 may be an enamelled copper wire, and the liquid conducting and heating body 131 may include an insulated layer formed on an outer peripheral surface.

When a high frequency alternating current flows along the inductive coil 132, a high frequency magnetic field (see dotted lines as shown in FIG. 3) is generated. Then a large quantity of eddy current is generated in the metallic powder of the liquid conducting and heating body 131 , so that the liquid conducting and heating body 131 heats up itself quickly. At the same time, the liquid conducting and heating body 131 absorbs tobacco liquid, and the tobacco liquid is heated by the liquid conducting and heating body 131 to form aerosol.

Since the liquid conducting and heating body 131 heats as a whole, the liquid conducting and heating body 131 has a large heating surface, and less loss of heat. Thus, a thermal efficiency of the liquid conducting and heating body 131 is high. Furthermore, because the metallic powder is evenly arranged in the liquid conducting and heating body 131, the liquid conducting and heating body 131 heats uniformly.

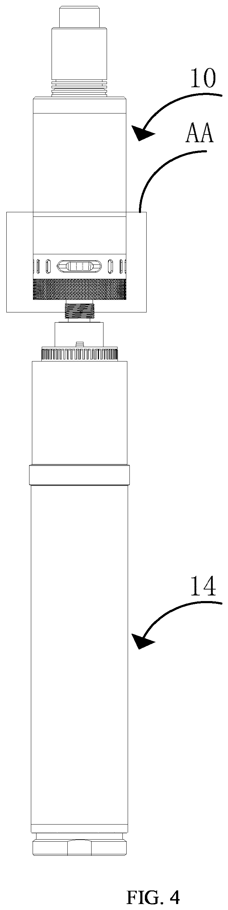

Referring to FIGS. 4-5 together, the electronic cigarette further includes an airflow adjusting ring 13 in the main body 11. The airflow adjusting ring 13 is configured for adjusting an output of the aerosol. The main body 11 defines an air inlet 113. The airflow adjusting ring 13 adjusts the output of the aerosol by controlling an opening dimension of the air inlet 113, thus satisfying demand of different users.

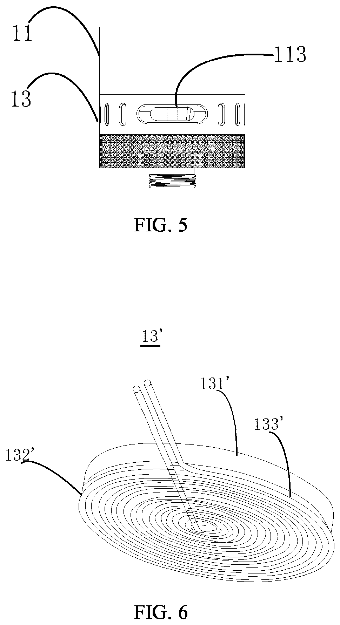

Referring to FIG. 6, a heating assembly 13' according to a second embodiment is shown. The heating assembly 13' includes a liquid conducting and heating body 131' and an inductive coil 132'.

The liquid conducting and heating body 131 is disk-shaped. The inductive coil 132' is a disk-shaped coil, which is substantially parallel to the liquid conducting and heating body 131. One end of the inductive coil 132' is led out from a center of the inductive coil 132', the other end of the inductive coil 132' is led out from a periphery of the inductive coil 132'. Two ends of the inductive coil 132' are connected to an alternating current supply. Quite usefully, the liquid conducting and heating body 131' and the inductive coil 132' have an identical shape.

In the present embodiment, the heating assembly 13' further includes an insulator 133' sandwiched between the liquid conducting and heating body 131' and the inductive coil 132'.

Working principle and manufacturing method of the heating assembly 13' are similar to those of the heating assembly 13, and will not be described for brevity.

The liquid conducting and heating body 131' and the inductive coil 132' are both disc-shaped. Accordingly, a magnetic line force generated by the inductive coil 132' is more concentrated. Therefore, the efficiency of the heating assembly 13' is higher than that of the first embodiment.

## Claims

1. A heating assembly (13, 13'), comprising:
a liquid conducting and heating body (131, 131'), the liquid conducting and heating body (131, 131') comprising a porous ceramic main body and a metallic powder; and
an inductive coil (132, 132') corresponding to the liquid conducting and heating body (131, 131');
wherein the inductive coil (132, 132') is configured for generating high frequency magnetic field in response to an alternating current, so that eddy current and heat are generated in the liquid conducting and heating body (131, 131'); the liquid conducting and heating body (131, 131') is configured for absorbing tobacco liquid and heating the tobacco liquid to form aerosol, **characterized in that** the metallic powder is evenly distributed in the ceramic main body, and **in that** the ceramic main body and the metallic powder is integrally formed.

2. The heating assembly (13) according to claim 1, wherein the liquid conducting and heating body (131) is cylindrical or tubular, and the inductive coil (132) is wound around an outer peripheral surface of the liquid conducting and heating body (131).

3. The heating assembly (13') according to claim 1, wherein the liquid conducting and heating body (131') is disk-shaped, the inductive coil (132') is a disk-shaped coil, and the inductive coil (132') is substantially parallel to the liquid conducting and heating body (131'); a first end of the inductive coil (132') is led out from a center of the inductive coil (132'), and an opposite second end of the inductive coil (132') is led out from a periphery of the inductive coil (132').

4. The heating assembly (13') according to claim 3, wherein the heating assembly (13') further comprises an insulator (133') sandwiched between the liquid conducting and heating body (131') and the inductive coil (132').

5. An electronic cigarette, comprising:
a main body (11);
a heating assembly (13, 13') according to any of claims 1-4 arranged in the main body (11); and
a power supply (14) configured for supplying the heating assembly (13, 13') with alternating current.

6. The electronic cigarette according to claim 5, further defining a liquid conducting groove (12) in communication with the liquid chamber (111), wherein the main body (11) defines a liquid chamber (111) and an air passage (112), the liquid chamber (111) is configured for storing tobacco liquid, the heating assembly (13) is arranged on the air passage (112), the liquid conducting and heating body (131) is in communication with the liquid chamber (111) via the liquid conducting groove (12), and the aerosol is expelled via the air passage (112).

7. The electronic cigarette according to claim 6, wherein the air passage (112) is oriented along an axial direction of the main body (11), the liquid conducting and heating body (131) is oriented substantially perpendicular to the air passage (112), an end of the liquid conducting and heating body (131) is plugged into the liquid conducting groove (12), and the end of the liquid conducting and heating body (131) contacts with the tobacco liquid in the liquid conducting groove (12).

8. The electronic cigarette according to claim 6, wherein the main body (11) defines an air inlet (113); the electronic cigarette further comprises an airflow adjusting ring (13) in the main body (11), and the airflow adjusting ring (13) is configured for adjusting an output of the aerosol by controlling an opening dimension of the air inlet (113).

9. An atomizer (10), comprising:
a housing;
a liquid chamber (111) defined in the housing configured for storing tobacco liquid; and
a heating assembly (13) according to any of claims 1-4 arranged in the housing.

## Patentansprüche

1. Eine Heizvorrichtung (13, 13'), umfassend:
einen Flüssigkeitsleit- und Heizkörper (131, 131'), wobei der Flüssigkeitsleit- und Heizkörper (131, 131') einen porösen Keramikhauptkörper und ein Metallpulver sowie
eine dem Flüssigkeitsleit- und Heizkörper (131, 131') zugeordnete Induktionsspule (132, 132');
wobei die Induktionsspule (132, 132') so ausgelegt ist, dass sie als Reaktion auf einen Wechselstrom ein hochfrequentes Magnetfeld erzeugt, sodass im Flüssigkeitsleit- und Heizkörper (131, 131') Wirbelströme und Wärme erzeugt werden; wobei der Flüssigkeitsleit- und Heizkörper (131, 131') dazu ausgelegt ist, Tabakflüssigkeit aufzunehmen und die Tabakflüssigkeit zu erhitzen, um Aerosol zu bilden, **dadurch gekennzeichnet, dass** das Metallpulver gleichmäßig im Keramikhauptkörper verteilt ist und dass der Keramikhauptkörper und das Metallpulver einstückig ausgebildet sind.

2. Die Heizbaugruppe (13) nach Anspruch 1, wobei der Flüssigkeitsleit- und Heizkörper (131) zylindrisch oder rohrförmig ist und die Induktionsspule (132) um eine Außenumfangsfläche des Flüssigkeitsleit- und Heizkörpers (131) gewickelt ist.

3. Die Heizbaugruppe (13') nach Anspruch 1, wobei der Flüssigkeitsleit- und Heizkörper (131') scheibenförmig ist, die Induktionsspule (132') eine scheibenförmige Spule ist und die Induktionsspule (132') im Wesentlichen parallel zum Flüssigkeitsleit- und Heizkörper (131') verläuft; ein erstes Ende der Induktionsspule (132') aus der Mitte der Induktionsspule (132') herausgeführt ist und ein gegenüberliegendes zweites Ende der Induktionsspule (132') aus dem Umfang der Induktionsspule (132') herausgeführt ist.

4. Die Heizbaugruppe (13') nach Anspruch 3, wobei die Heizbaugruppe (13') ferner einen Isolator (133') umfasst, der zwischen dem Flüssigkeitsleit- und Heizkörper (131') und der Induktionsspule (132') angeordnet ist.

5. Eine elektronische Zigarette, umfassend:
einen Hauptkörper (11);
eine Heizeinheit (13, 13') gemäß einem der Ansprüche 1 bis 4, die im Hauptkörper (11) angeordnet ist; und
eine Stromversorgung (14), die dazu ausgelegt ist, die Heizbaugruppe (13, 13') mit Wechselstrom zu versorgen.

6. Elektronische Zigarette nach Anspruch 5, die ferner eine mit der Flüssigkeitskammer (111) in Verbindung stehende Flüssigkeitsleitrille (12) aufweist, wobei der Hauptkörper (11) eine Flüssigkeitskammer (111) und einen Luftkanal (112) aufweist, die Flüssigkeitskammer (111) zur Aufnahme von Tabakflüssigkeit ausgelegt ist, die Heizbaugruppe (13) am Luftkanal (112) angeordnet ist, der Flüssigkeitsleit- und Heizkörper (131) über die Flüssigkeitsleitrille (12) mit der Flüssigkeitskammer (111) in Verbindung steht und das Aerosol über den Luftkanal (112) ausgestoßen wird.

7. Elektronische Zigarette nach Anspruch 6, wobei der Luftkanal (112) entlang einer axialen Richtung des Hauptkörpers (11) ausgerichtet ist, der Flüssigkeitsleit- und Heizkörper (131) im Wesentlichen senkrecht zum Luftkanal (112) ausgerichtet ist, ein Ende des Flüssigkeitsleit- und Heizkörpers (131) in die Flüssigkeitsleitrille (12) eingesteckt ist und das Ende des Flüssigkeitsleit- und Heizkörpers (131) mit der Tabakflüssigkeit in der Flüssigkeitsleitrille (12) in Kontakt steht.

8. Elektronische Zigarette nach Anspruch 6, wobei der Hauptkörper (11) einen Lufteinlass (113) definiert; die elektronische Zigarette ferner einen Luftstrom-Einstellring (13) im Hauptkörper (11) umfasst und der Luftstrom-Einstellring (13) dazu ausgelegt ist, die Aerosolabgabe durch Steuerung der Öffnungsgröße des Lufteinlasses (113) einzustellen.

9. Ein Zerstäuber (10), umfassend:
ein Gehäuse;
eine im Gehäuse ausgebildete Flüssigkeitskammer (111), die zur Aufnahme von Tabakflüssigkeit ausgelegt ist; und
eine Heizbaugruppe (13) gemäß einem der Ansprüche 1 bis 4, die in dem Gehäuse angeordnet ist.

## Revendications

1. Ensemble de chauffage (13, 13'), comprenant :
un corps de conduction et de chauffage de liquide (131, 131'), ledit corps de conduction et de chauffage de liquide (131, 131') comprenant un corps principal en céramique poreuse, une poudre métallique et
une bobine inductive (132, 132') correspondant au corps de conduction et de chauffage de liquide (131, 131') ;
dans lequel la bobine inductive (132, 132') est configurée pour générer un champ magnétique à haute fréquence en réponse à un courant alternatif, de sorte que des courants de Foucault et de la chaleur soient générés dans le corps de conduction et de chauffage de liquide (131, 131') ; le corps de conduction et de chauffage du liquide (131, 131') est configuré pour absorber le liquide de tabac et chauffer ce dernier afin de former un aérosol, **caractérisé en ce que** la poudre métallique est répartie uniformément dans le corps principal en céramique et **en ce que** le corps principal en céramique et la poudre métallique sont formés d'un seul tenant.

2. Ensemble de chauffage (13) selon la revendication 1, dans lequel le corps de conduction et de chauffage du liquide (131) est cylindrique ou tubulaire, et la bobine inductive (132) est enroulée autour d'une surface périphérique extérieure du corps de conduction et de chauffage du liquide (131).

3. Ensemble de chauffage (13') selon la revendication 1, dans lequel le corps de conduction et de chauffage du liquide (131') est en forme de disque, la bobine inductive (132') est une bobine en forme de disque, et la bobine inductive (132') est sensiblement parallèle au corps de conduction et de chauffage du liquide (131') ; une première extrémité de la bobine inductive (132') sort du centre de la bobine inductive (132'), et une deuxième extrémité opposée de la bobine inductive (132') sort de la périphérie de la bobine inductive (132').

4. Ensemble de chauffage (13') selon la revendication 3, dans lequel l'ensemble de chauffage (13') comprend en outre un isolant (133') intercalé entre le corps de conduction et de chauffage du liquide (131') et la bobine inductive (132').

5. Cigarette électronique, comprenant :
un corps principal (11) ;
un ensemble de chauffage (13, 13') selon l'une quelconque des revendications 1 à 4, disposé dans le corps principal (11) ; et
une alimentation électrique (14) configurée pour alimenter l'ensemble de chauffage (13, 13') en courant alternatif.

6. Cigarette électronique selon la revendication 5, définissant en outre une rainure de circulation de liquide (12) en communication avec la chambre à liquide (111), dans laquelle le corps principal (11) définit une chambre à liquide (111) et un passage d'air (112), la chambre à liquide (111) est configurée pour stocker du liquide de tabac, l'ensemble de chauffage (13) est disposé sur le passage d'air (112), le corps de conduction de liquide et de chauffage (131) est en communication avec la chambre à liquide (111) via la rainure de conduction de liquide (12), et l'aérosol est expulsé via le passage d'air (112).

7. Cigarette électronique selon la revendication 6, dans laquelle le passage d'air (112) est orienté selon une direction axiale du corps principal (11), le corps de conduction et de chauffage du liquide (131) est orienté sensiblement perpendiculairement au passage d'air (112), une extrémité du corps de conduction et de chauffage du liquide (131) est insérée dans la rainure de conduction du liquide (12), et l'extrémité du corps de conduction et de chauffage du liquide (131) est en contact avec le liquide de tabac dans la rainure de conduction du liquide (12).

8. Cigarette électronique selon la revendication 6, dans laquelle le corps principal (11) définit une entrée d'air (113) ; la cigarette électronique comprend en outre une bague de réglage du débit d'air (13) dans le corps principal (11), et la bague de réglage du débit d'air (13) est configurée pour régler le débit d'aérosol en contrôlant la dimension de l'ouverture de l'entrée d'air (113).

9. Atomiseur (10), comprenant :
un boîtier ;
une chambre à liquide (111) définie dans le boîtier et configurée pour stocker du liquide de tabac ; et
un ensemble de chauffage (13) selon l'une quelconque des revendications 1 à 4, disposé dans le boîtier.
